# EUROPEAN PATENT APPLICATION

(11) **EP 1 683 517 A1**
(43) Date of publication of application: **26.07.2006**
(21) Application number: 06007715.3
(22) Date of filing: 19.08.1997
(51) Int. Cl.: A61K 9/50, A61K 47/42

(54) **Methods for the production of protein particles useful for delivery of pharmacological agents**

(30) Priority: 19.08.1996 US 23968 P
(62) Divisional of application: 97936517.8
(71) Applicant: American Bioscience, Inc., Santa Monica, CA 90403 (US)
(72) Inventor: Magdassi, Shlomo, 96626 Jerusalem (IL); Desai, Neil, Los Angeles CA 90066 (US); Ferreri, Kevin, Los Angeles CA 90066 (US); Soon-Shiong, Patrick, Los Angeles CA 90049 (US)
(74) Representative: Roques, Sarah Elizabeth

(57) **Abstract**

A method has been developed for the formation of submicron particles (nanoparticles) by heat-denaturation of proteins (such as human serum albumin) in the presence of multivalent ions (such as calcium). Also provided are novel products produced by the invention method. An appropriate concentration of multivalent ions, within a relatively narrow range of concentrations, induces the precipitation of protein in the form of colloidal particles, at a temperature which is well below the heat denaturation temperature of the protein (as low as 60°C for serum albumin). Temperatures at which invention method operates are sufficiently low to permit incorporation of other molecules (e.g., by co-precipitation), into submicron particles according to the invention, including compounds which cannot withstand high temperatures. Invention methods facilitate the production of protein nanoparticles and microparticles containing various molecules (such as nucleic acids, oligonucleotides, polynucleotides, DNA, RNA, polysaccharides, ribozymes, pharmacologically active compounds, and the like) useful for therapeutic, diagnostic and other purposes. The addition of multivalent cations serves both to induce precipitation, and to allow linking of negatively charged molecules, such as DNA, to the negatively charged protein.

## Description

### FIELD OF THE INVENTION

The present invention relates to methods for the production of protein-based colloidal particulate vehicles, and compositions produced thereby. In a particular aspect, the present invention provides a novel procedure for the preparation of a colloidal system of proteins such as albumin and other molecules in the form of microparticles and nanoparticles, in the presence of divalent cations such as calcium.

### BACKGROUND OF THE INVENTION

Intravenous drug delivery permits rapid and direct equilibration with the blood stream, which then carries the medication to the rest of the body. It is desirable, however, to avoid the peak serum levels which are achieved within a short time after intravascular injection. A solution to this problem is to administer drugs carried within stable carriers, which would allow gradual release of the drugs inside the intravascular compartment following a bolus intravenous injection of therapeutic nanoparticles.

Injectable controlled-release nanoparticles can provide pre-programmed duration of action ranging from days to weeks to months from a single injection. Such nanoparticles can also offer several profound advantages over conventionally admininstered medicaments, such as, for example, automatic assured patient compliance with the dose regimen, as well as drug targeting to specific tissues or organs (see, for example, Tice and Gilley, in Journal of Controlled Release 2:343-352 (1985)). Use of submicron size microspheres (nanospheres) minimizes the incidence of pulmonary embolism often encountered with particles greater than 7 microns or particles which aggregate upon in vivo administration (see, for example, Gupta, et al., in International Journal of Pharmaceutics 43:167-177 (1988)).

Particulate microspheres facilitate the delivery of drugs to specific organs of the body. For example, a major aim in cancer chemotherapy is to improve the efficiency of cytostatic treatment. Killing of sensitive tumor cells is facilitated by exposing the cancerous lesions to high concentrations of anticancer drug, but conventional methods of administration prohibit the use of very high doses of the drugs due to their toxic secondary effects. However, intravenous delivery of drug-containing microspheres can prolong the serum half-life and bioavailability of the drug (see, for example, Leucuta, et al., in International Journal of Pharmaceutics 41:213-217 (1988)).

Since albumin microspheres have been used extensively in the diagnosis of reticuloendothelial abnormalities and in the measurement of blood flow, it has been desirable to develop albumin microspheres for active as well as passive drug targeting (see, for example, Gupta, et al., supra). An important prerequisite for clinical use of drug-containing microspheres is a stable product with reproducible characteristics. Microspheres were originally manufactured in suspension form for use in patients within a few hours, but techniques such as freeze-drying have been employed to increase the shelf-life of the product. For example, albumin microspheres have been prepared by formation of water-in-oil emlusions, where the droplets contain a protein and a drug, and the droplets are further crosslinked by covalent bonds by using a crosslinking agent such as glutaraldehyde. This method is capable of yielding suspensions of drug-loaded albumin microspheres in aqueous buffers for intravascular administration (see, for example, Willmott and Harrison, in International Journal of Pharmaceutics 43:161-166 (1988)).

Heat-denaturation of albumin to form drug-containing microspheres is often preferred to the use of crosslinking agents such as glutaraldehyde, because heat-denaturation avoids potentially undesirable chemical crosslinking reactions involving the therapeutic drug itself. For example, cotton seed oil has been added to a solution of bovine serum albumin, and then ultrasonicated to obtain a water-in-oil emulsion. This emulsion is then added dropwise to a larger volume of rapidly stirred cottonseed oil which has been preheated to temperatures between 105°C and 150°C. Such high temperatures are needed to achieve denaturation and precipitation of the serum albumin from the microdroplets of the emulsion, thereby forming stabilized microspheres which can be washed and dried to obtain a free-flowing powder of drug-containing microspheres (see, for example, Gupta, et al., supra).

US Patent No. 5,041,292, issued to J. Feijen, also discloses a process for preparation of microspheres, by forming a water-in-oil emulsion of olive oil and an aqueous solution of bovine serum albumin and a polysaccharide such as Heparin, and then heating the emulsion to 100-170°C, or adding a crosslinking agent such as glutaraldehyde. The microspheres are loaded later with a suitable pharmacologically active agent.

US Patent No. 5,133,908, assigned to S. Stainmesse, describes a method to form nanoparticles by dissolving the protein, e.g., serum albumin, at low temperature, and at a pH far above or below the isoelectric point, and adding this solution into a larger volume of water at very high temperature (e.g., boiling).

EP Application No. 349,428, by Devissaguet et al., describes a method for producing colloidal suspensions of spherical protein particles employing the following steps:
a. dissolving the protein and optionally an active agent in aqueous medium below the coagulation temperature of the protein, optionally with added surfactant,
b. heating an aqueous medium, optionally containing biologically active substance and surfactant (s), to a temperature above the coagulation temperature of the protein, and
c. adding the second solution to the first solution, under moderate agitation at a pH far from the isoelectric point of the protein.

Unfortunately, some drugs are destabilized or inactivated by the conditions typically used to obtain heat-denatured microspheres. It is therefore highly desirable to develop a general method for manufacturing drug-containing protein microspheres by heat denaturation which can be carried out at temperatures low enough to prevent drug inactivation, but which is still capable of producing an effective and stable carrier product with well controlled uniformity of particle sizes.

The formation of nanomatrixes of proteins has recently been reported (see US Patent No. 5,308,620, by R.C.K. Chen, issued in 1994). The method is based on mixing at least two types of proteins (hemoglobin and albumin), and addition of a solution containing organic solvent (e.g., an alcohol), without heat treatment. The resulting system is a turbid suspension of monodispersed nanomatrixes which are typically larger than 1 micron but less than 4 microns in diameter.

US Patent No. 5,049,322, issued to Devissaguet et al., discloses a method of producing a colloidal system containing 150-450 nm particles by dissolving a single protein in a solvent and adding ethanol or ethanol-containing surfactant. These particles have a core material which is encapsulated inside a wall material made of the protein.

In spite of these recent developments, there is still a need in the art for a general method for manufacturing drug-containing protein microspheres without the need for organic solvents and/or surfactants. Especially desirable would be methods for manufacturing drug-containing protein microspheres by heat denaturation at temperatures low enough to prevent drug inactivation, but which is still capable of producing an effective and stable carrier product with well controlled uniformity of particle sizes.

### OBJECTS OF THE INVENTION

It is an object of the present invention to provide a method for the formation of particles by heat-denaturation of proteins (e.g., human serum albumin), at a pH above the iso-electric point without the need for organic solvents and/or surfactants.

This and other objects of the invention will become apparent to those of skill in the art upon review of the specification and appended claims.

### BRIEF DESCRIPTION OF THE INVENTION

In accordance with the present invention, we have discovered that stable protein particles can be produced by heat-denaturation of proteins (e.g., human serum albumin), at a pH above the iso-electric point in the presence of an appropriate concentration of polyvalent cations (e.g., calcium ions) at temperatures below the heat denaturation temperature of the protein. Heating of the protein solution in the presence of polyvalent (e.g., calcium) cations allows co-precipitation of other negatively charged molecules such as hemoglobin, antibodies (at pH above the isoelectric point) and DNA or pharmacologically active molecules (including uncharged molecules) which cannot withstand high temperatures.

The addition of multivalent ions (such as calcium) leads to precipitation of protein particles by heat treatment at temperatures which are significantly lower than the denaturation temperature of the protein. In accordance with the present invention, it has been discovered that this ability of polyvalent cations to promote the precipitation of protein particles occurs only within a very narrow range of electrolyte concentrations.

Thus, the present invention relates to performing a heat induced precipitation, which, in the presence of divalent ions (e.g., calcium) at a specific concentration range, forms submicron particles of albumin or particles of albumin and other molecules such as DNA. The heat denaturation takes place at temperatures significantly below that of the protein in the absence of the divalent cations. In addition, the protein particles form without the need for formation of a water-in-oil emulsion, or addition of any organic solvent, cross-linking agent or surfactants, at the defined cation concentration range.

Thus, the present invention provides a method for the formation of submicron particles (nanoparticles) by heat-denaturation of proteins (e.g., human serum albumin) in the presence of multivalent ions (e.g., calcium). The use of an appropriate concentration of cations (e.g., calcium ions) induces the precipitation of the protein at a temperature which is significantly lower than the regular heat denaturation temperature of the protein. The process also allows co-precipitation of other molecules (including those which cannot withstand high temperatures), which are then incorporated within the nanoparticles. This procedure facilitates the production of nanoparticles containing various proteins, pharmacologically active agents or DNA for various purposes such as drug delivery, diagnostics, gene therapy, and the like. The present invention ultimately yields a colloidal system in the form of particles composed of proteins and other active molecules, having a diameter of several micrometers down to about 50 nm, depending on the specific cation and protein used, the concentrations of each, pH and temperature.

### DETAILED DESCRIPTION OF THE INVENTION

In accordance with the present invention, there are provided methods for the preparation of protein particles having a diameter less than about 30 microns. Invention methods comprise:
dissolving at least one protein, and optionally other non-proteinaceous materials, in an aqueous solution containing an amount of at least one multivalent cation sufficient to promote the formation of insoluble particles at a temperature below that of the heat denaturation temperature of said protein in purified form,
   wherein said aqueous solution is substantially free of organic media, and
heating the solution to a temperature below that of the heat denaturation temperature of an aqueous solution of said protein in purified form for a period of time sufficient to form insoluble particles containing the protein and optional non-proteinaceous materials.

Invention methods can readily be carried out in the substantial absence of organic solvent, i.e., organic solvent is not required to facilitate the invention method. Avoidance of organic solvent substantially reduces the possibility for denaturation of sensitive pharmacologically active agents as a result of the microparticle-forming process.

Proteins contemplated for use in the practice of the present invention include structural proteins, enzymes, antibodies, peptides, and the like. Specific proteins contemplated for use herein include albumins, collagen, gelatin, immunoglobulins, insulin, hemoglobin, transferrin, caesins, pepsin, trypsin, chymotrypsin, lysozyme, *α*-2-macroglobulin, fibronectin, vitronectin, fibrinogen, laminin, lipase, interleukin-1, interleukin-2, tissue necrosis factor, colony-stimulating factor, epidermal growth factor, transforming growth factors, fibroblast growth factor, insulin-like growth factors, hirudin, tissue plasminogen activator, urokinase, streptokinase, erythropoietin, Factor VIII, Factor IX, insulin, somatostatin, proinsulin, macrophage-inhibiting factor, macrophage-activating factor, muramyl dipeptide, interferons, glucocerebrosidase, calcitonin, oxytocin, growth hormone, *α*-1 antitrypsin, superoxide dismutase (SOD), catalase, adenosine deaminase, lactalbumin, ovalalbumin, amylase, and the like.

Optional non-proteinaceous materials contemplated for use herein include nucleic acids, oligonucleotides, polynucleotides, DNA, RNA, polysaccharides, ribozymes, pharmacologically active compounds capable of inclusion within the protein particles, and the like.

Nucleic acids contemplated for use in the practice of the present invention include sense or antisense nucleic acids encoding (or complementary to nucleic acids encoding) any protein suitable for delivery by inhalation.

DNA introduced as part of invention particles can be used for a variety of purposes, e.g., for gene delivery, for cell transfection, and the like.

Polysaccharides contemplated for use in the practice of the present invention include starches, celluloses, dextrans, alginates, chitosans, pectins, hyaluronic acid, and the like.

Pharmacologically active compounds capable of inclusion within the protein particles contemplated for incorporation into invention particles include antisense nucleic acids, antiviral compounds, anticancer agents, immunosuppressive agents, and the like. Exemplary pharmacologically active agents contemplated for use herein include antiviral agents (e.g., interferon gamma, zidovudine, amantadine hydrochloride, ribavirin, acyclovir, and the like), anticancer agents, immunosuppressants (e.g., glucocorticoids, buspirone, castanospermine, ebselen, edelfosine, enlimomab, galaptin, methoxatone, mizoribine), and the like.

Multivalent cations contemplated for use in the practice of the present invention include calcium, zinc, magnesium, barium, copper, iron, manganese, nickel, aluminium, gadolinium, technecium, strontium, cobalt, and the like. Isotopes of these elements are also contemplated for potential use in such applications as diagnostics and radionuclide therapy.

In accordance with the present invention, multivalent cations are introduced in an amount sufficient to promote the formation of insoluble particles at a temperature below that of the heat denaturation temperature of said protein in purified form. Typically, the concentration of multivalent cations falls in the range of about 0.1 mM up to about 10 mM. This concentration range depends on the ion type used, the pH of the protein-containing solution, the presence of solvent(s), and the like.

In accordance with the present invention, suitable protein(s), optional non-proteinaceous materials, and multivalent cation(s) are combined in a suitable aqueous solution. As readily recognized by those of skill in the art, suitable solutions for such purpose include distilled water, deionized water, buffered aqueous media, solutions of water and water-miscible solvent(s), and the like.

In accordance with the present invention, once the combination of protein(s), optional non-proteinaceous materials, and multivalent cation(s) have been combined in a suitable aqueous solution, the combination is subjected to heat to a temperature below that of the heat denaturation temperature of an aqueous solution of said protein in purified form for a period of time sufficient to form insoluble particles containing the protein and optional non-proteinaceous materials. Such heating is typically carried out at a temperature less than about 100°C. It is presently preferred that such heating be carried out at a temperature less than about 80°C.

A detailed understanding of the thermal properties of proteins (e.g., albumin) has been essential in the development of the invention process. In particular, conventional preparation of human serum albumin for clinical purposes involves treatment at 60°C for about 10 hours to inactivate the hepatitis virus. In the absence of multivalent ions, little denaturation or aggregation occurs at 60°C and the native albumin structure is largely retained, even without a stabilizer. However, when the temperature is kept above 80°C, about 40% of the albumin becomes irreversibly denatured and a considerable degree of aggregation is observed. The formation of intermolecular disulfide bridges by free sulfhydryl groups plays an essential role in the aggregation process (see, for example, Wetzel, et al., European Journal of Biochemistry 104:469-478 (1980)).

Suitable pHs contemplated for use in the invention process are in the range 1.5 - 11. Electrolyte concentration in the aqueous medium containing the protein, peptide, nucleic acids, pharmacological agents is in the range 0.01 mM - 1.0 M.

A typical process is based on preparation of the protein solution at pH above the isoelectric point (e.g., 1% serum albumin at pH 6.4), together with multivalent cation (e.g., calcium) at a defined, very low concentration, and optionally a non-proteinaceous material (i.e., an active molecule such as DNA), followed by heating the solution at a temperature below the usual heat denaturation temperature (for example, serum albumin should be heated to about 60°C). The duration of heating, the temperature, and the concentration of the multivalent ions will determine the average size of the particles, and their concentration in solution.

The method of the present invention does not require formation of a water-in-oil emulsion and heating to very high temperatures, nor addition of crosslinking agents, as commonly described in other patents and publications.

In addition to the formation of solid particles, hollow particles comprising a shell of proteinaceous material may be formed by dispersion of gas into the aqueous medium during the process of formation of the particles. The invention process results in a lower formation temperature for the hollow particles such as described in US Patent No. 4,957,656 (Cerny et al., 1990).

In accordance with another embodiment of the present invention, there are provided products produced by the above-described methods. Such products are useful for a variety of purposes, e.g., controlled release of a variety of pharmacologically active agents, for protected delivery of sensitive reagents (e.g., DNA, peptides, enzymes, and the like), and the like.

In accordance with yet another embodiment of the present invention, there are provided methods for the controlled release delivery of pharmacologically active agents to a patient in need thereof, said method comprising administering to said patient an effective amount of said pharmacologically active agent incorporated into a product as described herein.

The invention will now be described in greater detail by reference to the following non-limiting examples.

### Example 1

### Formation of microparticles and nanoparticles from albumin in the presence of divalent cations

Human serum albumin was dissolved in deionized, sterile water, to yield a clear 1% w/w stock albumin solution, having a pH 6.3, which is above the isoelectric point of the protein. The stock solution was used for the following experiments:
A) Three ml of this solution was immersed into a water bath at 65°C, for 5 minutes. The solution remained clear, and no particles were observed by light microscope.
B) Two ml of the stock solution were mixed with 0.08 ml of 100 mM calcium chloride solution, and 0.92 ml sterile water (final calcium chloride concentration 2.66 mM). The solution was immersed into a water bath, at 65°C, for 5 minutes. The solution became cloudy about two minutes after immersion. When viewed by light microscopy (magnification x 1000), particles, having a size 1 *µ*m and below were observed. The average size of these particles (conducted by a Malvern light scattering instrument), proved that these are albumin submicron particles, having a Z-average diameter of 460 nm. The particles were negatively charged, and have a Zeta-potential of -10 mv (measured by Malvern Zetasizer). This colloidal system was stable for at least two weeks at 4°C, without any precipitation. A frozen sample was found to retain its initial size, after thawing. The stability of the particles was also tested by centrifugation, washing and redispersion in sterile water: it was found that the particles were resuspended to give a colloidal system which contains particles having a diameter 1 *µ*m and below.
C) A similar experiment was conducted, in which the final calcium chloride concentration is 13.3 mM, or 6.6 mM. In both cases, larger particles, in the micron-size range (up to 50 *µ*m) were observed. These particles were aggregates of smaller particles, as observed by light microscopy.

These experiments indicate that at a given temperature and incubation time, colloidal particles of albumin can be formed if calcium chloride is present at very low concentration, and that submicron particles can be formed only within a very narrow range of calcium chloride concentrations.

### Example 2

### Effect of calcium concentration

A series of solutions was prepared in which the concentration of human serum albumin remained constant, 0.66 % w/v, and the concentrations of calcium chloride were in the range of 0 - 20 mM. Each solution was heated in a water bath at 60°C for 2 minutes, and than was observed by an optical microscope. It was found that the solution without calcium chloride added, and the solutions which have calcium chloride at concentrations of 5.33 - 20 mM, were clear, and no particles were detected. However, only at a specific, narrow range of calcium chloride concentrations (i.e., 0.67 - 2.7 mM), the solutions were turbid, indicating the presence of a colloidal system. Microscopic evaluation revealed that particles having a size below 2.5 um were present in these turbid systems.

A similar experiment was performed at 65°C, with similar results. Thus, the albumin particles were formed only at a specific, narrow range of calcium chloride concentrations.

However, it was found that at the higher temperature, the calcium concentration range in which the albumin particles are formed is slightly extended, up to 6.7 mM. Above this calcium chloride concentration, the solutions remained clear.

This experiment indicates that the formation of protein particles is not a result of a simple salting - out precipitation process, and that, surprisingly, at high calcium concentration, colloidal particles are not formed, as would be expected from a salting-out process.

### Example 3

### Formation of DNA-Albumin colloidal particles

The purpose of this example is to demonstrate the formation of microparticles and nanoparticles which contain both a protein and DNA.

### DNA PREPARATION

The RSV-ßGal reporter plasmid was introduced into Escherichia coli JM109 cells by calcium chloride transformation (see, for example, Sambrook et al. in Molecular Cloning: A Laboratory Manual, 2nd ed. Vol. 1. 1989, Plainview, NY: Cold Spring Harbor Laboratory Press. The cells were grown from a single colony in 1 liter LB Broth (GibcoBRL #12780-029) containing 50 (g/ml ampicillin to an optical density of 1.32 at 600 nm. The plasmid was purified using the QIAGEN Plasmid Mega Kit (QIAGEN #12181) according to the manufacturer's instructions. The final yield was 2 ml RSV-ßGal plasmid at a concentration of 1.24 g/l in TE (10 mM Tris-HCl, 1 mM EDTA pH 8.0) . 806 *µ*l of the sample was diluted to 1 ml with TE to give a final concentration of 1 g/l, and this sample was divided into 50 µl aliquots for nanoparticle formation studies.

### FORMATION OF HSA-DNA PARTICLES

a. 1 ml HSA 1.25% w/w solution was mixed with 0.41 ml deionized water, 0.04 ml of 100 mM calcium chloride solution (to yield a final calcium chloride concentration of 2.66 mM) , 0.41 ml sterile deionized water and 0.05 ml DNA solution (1 mg/ml). The solution was immersed in a water bath (60°C) and after about one minute, the solution becomes turbid. The solution is removed from the water bath after about 2 minutes. Microscopic observation reveals the presence of particles having a size of 1 *µ*m and below. Light scattering measurments proved the presence of one population of nanoparticles, having a Z-average diameter of 420 nm.
b. larger HSA-DNA particles, having a size about 10 um, were prepared by the same procedure but the temperature was 65°C, while the heating period was only one minute.

### ANALYSIS OF HSA - DNA NANOPARTICLES

In the present study, two classes of HSA-DNA particles were made in the micron and submicron size ranges. The presence of DNA in both classes of colloidal particles was determined by two independent methods:

Following formation, the particles were separated from the supernatant by centrifugation and the two fractions collected and analyzed in parallel. Equivalent aliquots of both the pellet and the supernatant fractions were digested for two hours at 60°C with Proteinase K (GibcoBRL #255530-049; 100 *µ*g/ml in 20 mM Tris-HCl, 20 mM EDTA, 0.5% sodium dodecylsulfate pH 7.5), extracted twice with phenol:chloroform:isoamyl alcohol (25:24:1 v/v), extracted once with chloroform, then precipitated with 2 vol. ethanol in the presence of 0.3 M sodium acetate, pH 5.2. The samples were then electrophoresed on a 0.7% agarose gel and stained with ethidium bromide to ascertain the presence and integrity of the DNA.

It was found that the vast majority (estimated greater than 90%) of the DNA was in the particle fraction of both classes of samples, i.e., microparticles (having a mean diameter of 10 *µ*m) and nanoparticles (having a mean diameter of 420 nm).

In contrast, very little DNA was associated with the pellet of the control samples in which no particles were formed. Thus, for example, in the "no heating" control sample, the same components as used for the preparation of invention particulate samples were combined, but not heated and therefore did not contain any particles. In the "adsorbed DNA" control sample, the DNA was added after the formation of particles with HSA alone, i.e., the HSA particles were formed prior to the addition of DNA. Furthermore, the position of the DNA bands indicates that the DNA in the particles is intact, and may be used to obtain specific biological activity.

Interestingly, the DNA could not be extracted from the particles using the standard phenol:chloroform:isoamyl alcohol (25:24:1 v/v) procedure alone, even after addition of both 10 mM EDTA and 1% sodium dodecylsulfate. Since most proteins are extracted from DNA by these procedures (see, for example, Sambrook et al., supra), this is indicative of very tight associations within the HSA-DNA complexes.

The second method employed to verify the presence of DNA was based on specific DNA staining. The particles were stained with 1 µg/ml Hoechst 33258 (a DNA specific dye) in 50 mM sodium phosphate pH 7.4/2M NaCl [Labarca], and then visualized with an Olympus IX70 fluorescent microscope.

It was found that the particles containing DNA fluoresced bright blue, while particles of HSA alone were not fluorescent.

These experiments demonstrate that the DNA was indeed substantially entrapped within the micro and nanoparticles, and not simply adsorbed on the surface of the particles, making it possible to use such colloidal particles for gene delivery.

### Example 4

### Formation of Protein Particles with multivalent ions other than calcium

The formation of protein particles during heating in the presence of calcium ions at a specific concentration can also be achieved by using other multivalent ions (e.g., barium, copper, magnesium, and the like) which are capable of reducing the heat denaturation temperature of the protein. The type and required concentration of the specific multivalent ion used should be evaluated according to the type of protein (and other optional molecules) present in the system.

### Example 5

### Formation of albumin - IgG colloidal particles

The purpose of this example is to demonstrate the possibility to form particles which contain more than one protein. Therefore, a fluorescently lablled monoclonal IgG (rabbit) was used in order to allow simple and quick evaluation of the presence of the antibody by fluorescence microscope. One ml human serum albumin, at pH 7.1 (phosphate buffer, 0.25mM), was mixed with 0.1 ml FTIC lablled IgG (Sigma F-4151), 0.04 ml calcium chloride 100 mM solution, and 0.36 ml deionized water. The solution was heated in a water bath, at 60°C, for 5 minutes. The turbid dispersion which was obtained contained aggregates of particles in the size range of 50-100 *µ*m, while each aggregate was composed of many small (about 2 *µ*m) particles. The experiment was conducted at prolonged heating, in order to obtain large particles which can be easily viewed by an optical microscope.

It was found that all particles were fluorescent, giving bright green light due to the FITC labelling. Taking into consideration that the concentration of HSA is much higher than the concentration of the IgG, (about two orders of magnitude in the final solution), it can be concluded that the particles contain both albumin and antibodies.

### Example 6

### Targeting of Protein particles

The above prepared albumin-antibody particles can be used to deliver a drug, diagnostic agent or radiopharmaceutical and target it to a specific site in the body by incorporating into the particles, a recognition molecule or molecules. A non-limiting list of such molecules include proteins, peptides, antibodies, sugars, polysaccharides and combinations thereof. For example, an antibody against a human mammary carcinoma (MX-1) can be incorporated into these particles which are then subsequently introduced into the blood stream of a cancer patient suffering from the disease in order to carry a payload of therapeutic drug or radioactive isotope to treat the disease, or a diagnostic marker to locate the extent of disease. These targeting agents can be incorporated into the particles during the process of formation or after the protein particles are formed.

### Example 7

### Gene therapy with protein-DNA particles

The above prepared protein-DNA particles can be used for gene therapy by delivering the intact DNA into cells, under such conditions that effective transfection can be achieved. To increase the efficacy of particle uptake, addition of agents such as glycocholic acid, polylysine, gelatin, phospholipids, calcium ions, glycerol, and the like, into a suspension of the protein-DNA particles or using techniques such as electroporation and the like can improve the uptake of particles by various cells. In addition, these particles can be also linked to molecules capable of targeting the particles to specific organs and tissues, cells or even to specific sites inside the cell.

### Example 8

### Other uses of protein particles

The present invention may be used in order to achieve small particles of proteins with specific biological activity. For example, hemoglobin particles can be formed by a similar process as described above for albumin. Hemoglobin particles can be used, for example, as a blood substitute, as a therapeutic to treat anemias, and the like. In addition, the present invention can be used in order to form protein shells around air bubbles, thus enabling their use as an echocontrast agent, for medical diagnostics.

While the invention has been described in detail with reference to certain preferred embodiments thereof, it will be understood that modifications and variations are within the spirit and scope of that which is described and claimed.

## Claims

1. A method for the preparation of protein particles having a diameter less than about 30 microns, said method comprising:
dissolving at least one protein, and optionally other non-proteinaceous materials, in a solution containing an amount of at least one multivalent cation sufficient to promote the formation of insoluble particles at a temperature below that of the heat denaturation temperature of said protein in purified form, and
heating the solution to a temperature below that of the heat denaturation temperature of an aqueous solution of said protein in purified form for a period of time sufficient to form insoluble particles containing the protein and optional non-proteinaceous materials.

2. A method according to claim 1 wherein said particles have a diameter of less than about 2 *µ*m.

3. A method according to claim 1 wherein said protein is a structural protein, an enzyme, an antibody, or a peptide.

4. A method according to claim 1 wherein said protein is albumin, collagen, gelatin, immunoglobulin, insulin, hemoglobin, transferrin, caesin, pepsin, trypsin, chymotrypsin, lysozyme, α-2-macroglobulin, fibronectin, vitronectin, fibrinogen, laminin, lipase, interleukin-1, interleukin-2, tissue necrosis factor, colony-stimulating factor, epidermal growth factor, transforming growth factors, fibroblast growth factor, insulin-like growth factors, hirudin, tissue plasminogen activator, urokinase, streptokinase, erythropoietin, Factor VIII, Factor IX, somatostatin, proinsulin, macrophage-inhibiting factor, macrophage-activating factor, muramyl dipeptide, interferons, glucocerebrosidase, calcitonin, oxytocin, growth hormone, *α*-1 antitrypsin, superoxide dismutase (SOD), catalase, adenosine deaminase, lactalbumin, ovalalbumin, amylase, hemoglobin or albumin.

5. A method according to claim 1 wherein said protein is albumin.

6. A method according to claim 1 wherein the multivalent cation is calcium, zinc, magnesium, barium, copper, iron, manganese, nickel, aluminium, gadolinium, technecium, strontium, cobalt, or mixtures of any two or more thereof.

7. A method according to claim 6 wherein the multivalent cation is calcium.

8. A method according to claim 7 wherein the concentration of calcium ions is between 0.1 mM and 10 mM.

9. A method according to claim 1 wherein the optional non-proteinaceous material is a nucleic acid, an oligonucleotide, a polynucleotide, DNA, RNA, a polysaccharide, a ribozyme or a pharmacologically active compound capable of inclusion within the protein particles.

10. A method according to claim 9 wherein said DNA is used for gene delivery and cell transfection.

11. A method according to claim 1 wherein the non-proteinaceous material is a pharmacologically active agent capable of inclusion within the protein particles.

12. A method according to claim 11 wherein said pharmacologically active agent is an antisense nucleic acid, an antiviral compound, an anticancer agent or an immunosuppressive agent.

13. A method according to claim 12 wherein said pharmacologically active agent is interferon gamma, zidovudine, amantadine hydrochloride, ribavirin, acyclovir, glucocorticoids, buspirone, castanospermine, ebselen, edelfosine, enlimomab, galaptin, methoxatone or mizoribine.

14. A method according to claim 1 wherein the heat denaturation is carried out at a temperature less than about 100°C.

15. The product produced by the method of claim 1.

16. A method for the controlled release delivery of drugs to a patient in need thereof, said method comprising administering to said patient an effective amount of said drug incorporated into a product according to claim 15.
